**Europäisches Patentamt**

**European Patent Office**

**Office européen des brevets**

(19)

(11) Veröffentlichungsnummer : **0 358 964 B1**

(12) **EUROPÄISCHE PATENTSCHRIFT**

(45) Veröffentlichungstag der Patentschrift :
**04.11.92 Patentblatt 92/45**

(21) Anmeldenummer : **89115052.6**

(22) Anmeldetag : **16.08.89**

(51) Int. Cl.⁵ : **A61M 5/165**

(54) Infusions und//oder Injektions-system mit Mehrkammer-Filter und Mehrkammer Anschlussleitung.

(30) Priorität : **24.08.88 DE 3828672**

(43) Veröffentlichungstag der Anmeldung :
**21.03.90 Patentblatt 90/12**

(45) Bekanntmachung des Hinweises auf die
Patenterteilung :
**04.11.92 Patentblatt 92/45**

(84) Benannte Vertragsstaaten :
**AT BE CH DE ES FR GB IT LI LU NL SE**

(56) Entgegenhaltungen :
**DE-A- 2 510 148**
**US-A- 4 326 957**

(73) Patentinhaber : **WEX FILTERTECHNIK GMBH**
**Industriestrasse 17 Postfach 1409**
**W-6442 Rotenburg a.d.F. (DE)**

(72) Erfinder : **Wex, Roland**
**Am Hang 28**
**W-3508 Melsungen (DE)**

(74) Vertreter : **Quermann, Helmut, Dipl.-Ing.**
**Postfach 6145 Gustav-Freytag-Strasse 25**
**W-6200 Wiesbaden (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

**Beschreibung**

Die Erfindung betrifft ein Infusions- und/oder Injektionssystem mit einem Filter sowie infusions- und/oder injektionsseitigen und patientenseitigen Anschlußleitungen. Derartige systeme sind z.B. aus der US-A-4326957 oder der DE-A-2 510 148 bekannt.

Bekannte Infusions- und/oder Injektionssysteme mit Filtern sind so konzipiert, daß dort, wo mehrere Infusionslösungen und Medikamente im Intensivstationsbereich zusammen appliziert werden, vor dem Infusionsfilter Baugruppen von Mehrwegestücken angebracht sind, die die Flüssigkeiten zusammenführen.

Bei der Vielzahl von hochwirksamen Medikamenten und Wirkstoffen aus den Lösungen, die heute den Patienten zugeführt werden, können, bei gleichzeitiger Applikation, chemische Interaktionen entstehen.

Bekannt sind Ausflockungen, die bei einem Einsatz von Filtern die Membran kurzfristig zusetzen, wodurch es zu Problemen während der Infusion kommt.

Zu Beginn einer Intensivbehandlung ist nicht immer klar, welche Infusionstherapie angewendet werden muß. Vielmehr muß sich der Arzt den momentanen Bedürfnissen des Patienten anpassen und kurzfristig reagieren. Um die oben beschriebenen Probleme zu vermeiden, wird deshalb sehr oft auf den Einsatz von Filtern verzichtet, obwohl dadurch die Gefahr der partikulären Verunreinigung und die Zuführung von Endotoxinen erhöht wird. Partikuläre Verunreinigungen in Lösungen mit Medikamenten und Einmalartikeln bedeuten ebenfalls eine erhebliche Patientengefährdung.

Es ist Aufgabe vorliegender Erfindung, ein Infusions- und/oder Injektionssystem mit einem Filter sowie infusions- und/oder injektionsseitigen und patientenseitigen Anschlußleitungen zu schaffen, das die Patientensicherheit bei der Zuführung von verschiedenen Infusionslösungen und Medikamenten durch Vermeidung frühzeitiger Vermischung vor dem Patienten wesentlich erhöht.

Die Lösung ist gekennzeichnet durch ein Filtergehäuse mit mehreren separaten Filterkammern, die jeweils ein Filterelement aufnehmen, ein dem Flüssigkeitseingang jeder Filterkammer zugeordnetes Anschlußelement zum Verbinden mit jeweils einer infusions- bzw. injektionsseitigen Anschlußleitung, sowie ein allen Filterkammern gemeinsames Anschlußelement zum Verbinden einer der Anzahl der Filterkammern entsprechenden mehrkanaligen, patientenseitigen Anschlußleitung mit den Flüssigkeitsausgängen der Filterkammern, wobei diese Anschlußleitung in ein einkanaliges patientennahes Anschlußstück mündet.

Das erfindungsgemäße Infusions- und/oder Injektionssystem ermöglicht es, durch das Filter gleichzeitig mehrere unterschiedliche Infusionslösungen und Medikamente mit unterschiedlichen Wirkstoffen und gleicher oder mit ungleicher Geschwindigkeit ohne Vermischung und somit Vermeidung von Interaktionen zu führen. Das Filtergehäuse kann dabei in verschiedenen geometrischen Ausführungen eine unterschiedliche Zahl von Filterkammern aufweisen, wobei jede Filterkammer einen eigenen Zulauf besitzt und die Filterkammern so gestaltet sind, daß sie einen gemeinsamen zentralen Auslaufpunkt aufweisen. Im Bereich dieses Auslaufpunktes wird die durch die jeweilige Filterkammer strömende Flüssigkeit unmittelbar einem Kanal der mehrkanaligen, patientenseitigen Anschlußleitung zugeführt, so daß die aus den Filterkammern austretenden Lösungen die patientenseitige Anschlußleitung ohne gegenseitige Vermischung durchströmen. Die Zahl der Filterkammern entspricht somit der Anzahl der Kanäle in dieser Anschlußleitung. Die Anschlußleitung mündet erst unmittelbar vor der Zuführung der Infusion bzw. Injektion in den Körper des Patienten in ein patientennahes Anschlußstück, um so chemische Interaktionen oder Ausflockungen zu verhindern.

Die Zahl und Größe der Filterkammern kann den verschiedenen Medikamenten- und Infusionslösunganforderungen angepaßt werden, somit unterschiedliche oder gleichartige Gewebe und/oder Membranmaterialien mit unterschiedlichen Porengrößen und Wirkungsarten aufweisen.

Gemäß einer besonderen Ausführungsform der Erfindung ist vorgesehen, daß die Filterkammern sich bezüglich des patientenseitigen Anschlußelementes jeweils über einen Kreissektor erstrecken und mit ihren benachbarten, die Flüssigkeitsausgänge aufweisenden Sektorspitzenbereichen in das patientenseitige Anschlußelement münden, wobei die patientenseitige Anschlußleitung entsprechend den Sektoren der Filterkammern in die einzelnen Kanäle unterteilt ist. Es wird dabei als besonders vorteilhaft angesehen, wenn das Filtergehäuse die Form eines Trapezes aufweist, das in drei identisch ausgebildete, die Form von Dreiecken aufweisende Filterkammern unterteilt ist, die unmittelbar aneinander angrenzend im Filtergehäuse angeordnet sind. Eine besonderes einfach gestaltete Filterkonfiguration ergibt sich, wenn die Dreiecke darüber hinaus als gleichseitige Dreiecke ausgebildet sind, womit sich der Filteraufbau symmetrisch zur Symmetrieachse des Trapezes gestalten läßt.

Eine besonders vorteilhafte Anwendung des Infusions- und/oder Injektionssystems läßt sich erzielen, wenn das Filtergehäuse plattenförmig ausgebildet und mit diesem eine sich senkrecht zur Filtergehäuse erstreckende Aufnahmeplatte verbunden ist. Es ist dabei anzustreben, daß die Auflageplatte während des Infusions- bzw. Injektionsvorganges horizontal zu liegen kommt, so daß das Filtergehäuse in dessen Plattenebene immer vertikal nach unten durchströmt wird. Vorteilhaft weist zudem das Filtergehäuse die Form eines Trapezes auf und ist die Auflageplatte mit der Grundseite des Trapezes verbunden. Bei größeren Infusions- bzw. Injektionsmengen können die Filterkammern mit Entlüftungsöffnungen und diesen zugeordneten Entlüftungsmembranen, insbesondere mit vor die Entlüftungsöffnungen gesetzten hydrophoben Membranen, versehen sein. Dabei sollten diese Entlüftungsöffnungen so konzipiert sein, daß sie an geometrisch günstigen Positionen mit bestmöglicher Entlüftungsfunktion angebracht sind. So können beispielsweise die Filterkammern die Form von Dreiecken aufweisen und jede Filterkammer im Bereich der beiden, der patientenseitigen Anschlußleitung abgewandten Ecken jeweils eine Entlüftungsöffnung mit Membran aufweisen.

Damit das Filter variabel im schwerkraft- und maschinellen Betrieb einsetzbar ist und sich ein gutes Flüssigkeitsdurchströmungsprinzip im Filtergehäuse einstellt, wird vorgeschlagen, daß das Filtergehäuse benachbart zur Auflageplatte das Anschlußelement zum Verbinden mit der patientenseitigen Anschlußleitung und beabstandet zur Auflageplatte die Anschlußelemente zum Verbinden mit dem infusions- bzw. injektionsseitigen Anschlußleitungen aufweist. Wird im vorbeschriebenen Sinne die Auflageplatte horizontal und zudem untenliegend plaziert, beispielsweise durch Befestigung am Bett oder mittels eines Pflasters am Patienten, können so die Flüssigkeiten von oben nach unten durch das Filter strömen. Als besonders vorteilhaft hat es sich in diesem Zusammenhang erwiesen, wenn die Anschlußelemente zum Verbinden mit den infusions- bzw. injektionsseitigen Anschlußleitungen in gleichem Abstand zur Auflageplatte angeordnet sind und damit gleiche Höhenniveaus zwischen den Anschlußelementen durchströmen müssen.

Vorteilhaft besteht das Filtergehäuse aus zwei in einer Ebene senkrecht zur Aufnahmeplatte geteilten Filtergehäusehälften, wobei eine der beiden Hälften die Anschlußelemente zum Verbinden mit dem infusions- und/oder injektionsseitigen Anschlußleitungen und die andere Hälfte das Anschlußelement zum Verbinden mit der patientenseitigen Anschlußleitung aufweist. Das Filtergehäuse sollte mit einem umlaufenden Randbereich versehen sein, so daß die Filtergehäusehälften für den Fall, daß sie aus Kunststoff bestehen, in diesem Bereich miteinander verschweißt werden können. Die Bildung der einzelnen Filterkammern erfolgt zweckmäßig durch an einem oder beiden Filtergehäusehälften angebrachte Trennstege, in deren Bereich die Filtergehäusehälften thermoplastisch verbunden sind, was die Druckstabilität der Gehäusehälften bedeutend verbessert und somit die Patienten- und Anwendungssicherheit erhöht.

Um das Durchflußverhalten der Lösungen zu optimieren und sicherzustellen, daß in den Lösungen befindliche Partikel und Schwebeteilchen die in den einzelnen Filterkammern befindlichen Filterelemente nicht vorzeitig zusetzen, ist vorgesehen, daß die den infusions- bzw. injektionsseitigen Anschlußelementen zugeordnete Filtergehäusehälfte im Bereich der Filterelemente eine diese umgebende umlaufende Nut aufweist und die Filtergehäusehälften Stützrippen für die Filterelemente besitzen, insbesondere Stützrippen, die senkrecht zur Ebene der Auflageplatte angeordnet sind. Es wird hierdurch gewährleistet, daß die Partikel und Schwebeteilchen über die zwischen den Stützrippen gebildeten senkrechtstehenden Flüssigkeitsnuten der Filterinnenseite in den jeweils unten befindlichen Abschnitt der umlaufenden Nut absinken können und sich dort ansammeln. Die umlaufende Nut wird darüber hinaus aber auch als Luft- oder Gasleitnut zu den Belüftungsflächen genutzt.

Die Verwendung des vorgeschlagenen Infusions- und/oder Injektionssystems mit der Filterkonstruktion eliminiert durch seine Vorteile das Problem der Interaktion vor dem Filter und erhöht dadurch die Patientensicherheit. Bekannte Spätfolgen aufgrund partikulärer und bakterieller Kontamination werden auf ein Minimum reduziert. Es können, wie dargestellt, verschiedene Filtergehäusekonfigurationen konzipiert werden. Dabei führt in jede Filterkammer ein Infusionszufluß. Dieser kann mengenabhängig größer oder kleiner gehalten werden. Die Filterkammern sind je nach Anwenderforderung mit unterschiedlichen Geweben oder Membranen mit unterschiedler Porengröße und somit Filtrationsleistung und Abscheidegrad auszustatten. Die Gehäusekonzeption ermöglicht klemm- oder thermoplastische Verbindungsverfahren der Gewebe oder Membranen. Die Filterkammern werden durch Trennstege dicht abgeschlossen, so daß die verschiedenen Medien nicht miteinander vermischt werden können. Die Anschlußelemente für den Infusions- bzw. Injektionszulauf können variabel als Lock-Anschlüsse oder Schlauchanschlüsse ausgestattet werden. Dadurch besteht die Möglichkeit, das mehrkanalige Filter als nachträglich einbaubares, einzeln steril verpacktes Filter oder als in Infusionsgeräte direkt integriertes Filter einzusetzen.

Weitere Merkmale der Erfindung sind in der Beschreibung der Figuren und in den Unteransprüchen dargestellt, wobei bemerkt wird, daß alle Einzelmerkmale und alle Kombinationen von Einzelmerkmalen weitere erfinderische Ausgestaltungen darstellen.

In den Figuren ist die Erfindung an einer Ausführungsform beispielsweise dargestellt, ohne auf diese beschränkt zu sein. Es stellt dar:

Figur 1 eine Draufsicht auf das erfindungsgemäße Infusions- und/oder Injektionssystem im Bereich des Filters und dessen Anschlußleitungen,

Figur 2 eine Ansicht A des in Figur 1 gezeigten Filters,

Figur 3 eine Ansicht B des in Figur 1 gezeigten Filters,

Figur 4 eine Seitenansicht des in Figur 1 gezeigten Filters mit den Anschlußleitungen,

Figur 5 eine Innenansicht einer Filtergehäusehälfte in Richtung der Pfeile C in Figur 4 gesehen,

Figur 6 eine Innenansicht der anderen Filtergehäusehälfte in Richtung der Pfeile D gemäß Figur 4 gesehen,

Figur 7 eine Seitenansicht des zum Infusions- und/oder Injektionssystem gehörenden patientennahen Anschlußstück mit der dazugehörigen Anschlußleitung und

Figur 8 einen Schnitt durch die patientenseitige Anschlußleitung gemäß der Linie E-E in Figur 4.

Das erfindungsgemäße Infusions- und/oder Injektionssystem besteht im wesentlichen aus einem Filter 1, dessen zweiteiliges Filtergehäuse 2 im Bereich einer Filtergehäusehälfte 3 drei Anschlußelemente 4, 5 und 6 zum Verbinden mit jeweils einer infusions- bzw. injektionsseitigen Anschlußleitung 7, 8 bzw. 9 aufweist und dessen andere Filtergehäusehälfte 10 ein Anschlußelement 11 zum Verbinden mit einer Anschlußleitung 12 besitzt, die in ein patientennahes Anschlußstück 13, das als Kanüle ausgebildet ist, mündet.

Im Detail ist das Filtergehäuse 2 plattenförmig ausgebildet und weist, wie insbesondere in den Figuren 2, 3 und 5, 6 verdeutlicht, die Form eines gleichschenkligen Trapezes auf. Die Figuren zeigen gleichfalls die Unterteilung des Filtergehäuses 2 mittels im Bereich der einander zugewandten Flächen der Filtergehäusehälften 3 und 10 angeordneter Trennstege 14, 15, 16 und 17 in drei Filterkammern 18, 19 und 20, die jeweils die Form eines gleichseitigen Dreiecks aufweisen. Durch diese Form bedingt erstrecken sich die Trennstege 14, 15, 16 und 17 ausgehend von dem Schnittpunkt 21 der Symmetrielinie 22 des Filtergehäuses 2 mit dessen Grundseite 23 zu den gegenüberliegenden Ecken 24 und 25 des Filtergehäuses 2, so daß unter Einbeziehung der im Bereich der Grundseite 23 gebildeten Ecken 26 und 27 des Filtergehäuses 2 die Filterkammer 18 durch den Punkt 21 und die Ecken 24 und 26, die Filterkammer 19 durch den Punkt 21 und die Ecken 24 und 25 sowie die Filterkammer 20 durch den Punkt 21 und die Ecken 25 und 27 gebildet sind.

Die Filterkammern 18, 19 und 20 nehmen deren Größe und Form angepaßte, nur schematisch angedeutete Filterelemente 28, 29 und 30 auf, die je nach Anwenderforderung unterschiedliche Gewebe aufweisen oder als Membranen mit unterschiedlicher Porengröße ausgeführt sind und damit für die einzelnen Filterkammern unterschiedliche Filtrationsleistungen und Abscheidegrade ermöglichen.

Das Filter 1 besteht aus einem thermoplastischen Kunststoff und es sind die Filtergehäusehälften 3 und 10 außen im Bereich deren einander zugewandten Flächen mit einer umlaufenden Filterklemmfläche 31 bzw. 32 versehen, die beim Aufeinanderfügen der Filtergehäusehälften 3 und 10 innen die einzelnen Filterelemente 28, 29 und 30 zwischen sich einklemmen und beim Verschweißen der Filterklemmflächen 31 und 32 im äußeren Bereich die Filterelemente 28, 29 und 30 dauerhaft zwischen sich fixieren, wobei die einzelnen Filterkammern 18, 19 und 20 innen durch die in Anlage miteinander gelangenden und gegebenenfalls miteinander thermoplastisch verbundenen Trennstege 14, 16 bzw. 15, 17 dicht abgeschlossen werden, so daß die verschiedenen, durch die Filterkammern strömenden Medien nicht miteinander vermischt werden können.

Wie der Darstellung der Figuren 2 und 6 zu entnehmen ist, weist die den Anschlußelementen 4, 5 und 6 zugeordnete Filtergehäusehälfte 3 Entlüftungsöffnungen 33 mit in dieser eingesetzten hydrophoben Entlüftungsmembranen 34 auf, die sich jeweils an einem in die Entlüftungsöffnung 33 eingesetzten Stützrippenkreuz 35 abstützen. So ist der Filterkammern 18 im Bereich der Ecken 26 und 24, der Filterkammer 19 im Bereich der Ecken 24 und 25 sowie der Filterkammer 20 im Bereich der Ecken 25 und 27 jeweils eine Entlüftungsöffnung 33 mit hydrophober Membran 34 und Stützrippenkreuz 35 zugeordnet.

Benachbart zu den in den Ecken 24 und 25 angeordneten Entlüftungsöffnungen 33 weist die Filtergehäusehälfte 3 symmetrisch zur Symmetrielinie 22 die Anschlußelemente 4 und 6 auf, ferner auf einer Verbindungslinie zwischen diesen beiden Anschlußelementen 4 und 6 auf der Symmetrielinie 22 das dritte Anschlußelement 5. Die zweite Filtergehäusehälfte 10 besitzt im Bereich des Schnittpunktes 21 der Trennstege 16 und 17 den Zugang zum Anschlußelement 11, der infolge der Trennstege 16 und 17 in drei Kanäle 36, 37 und 38 unterteilt ist. Beide Filtergehäusehälften 3 und 10 sind im Bereich ihrer die Filterelemente 28, 29 und 30 aufnehmenden Flächen mit parallel zur Symmetrielinie 22 verlaufenden Flüssigkeitsnuten 39 versehen. Die Filtergehäusehälfte 3 weist darüber hinaus zwischen den Anschlußelementen 4 und 6 und der der Grundseite 23 zugeordneten Filterklemmfläche 32 parallel zur Symmetrielinie 22 verlaufende, verbreiterte Flüssigkeitsnuten 40 auf.

Wie insbesondere der Darstellung der Figur 8 zu entnehmen ist, weist die mit dem patientenseitigen Anschlußelement 11 verbindbare Anschlußleitung 12 entsprechend der Sektoren der Filtergehäusehälfte 10 im Bereich des Schnittpunktes 21 drei Kanäle 44, 45 und 46 auf, wobei bei in das Anschlußelement 11 eingeschobener Stellung der Anschlußleitung 12 die diesem zugeordneten Bereiche der Trennstege 16 und 17 in Anlage mit den Stegen 47 und 48 gelangen, womit eine flüssigkeitsdichte Verbindung von der Filterkammer 18 in den Kanal 44, von der Filterkammer 19 in den Kanal 45 und von der Filterkammer 20 in den Kanal 46 der patientenseitigen Anschlußleitung 12 sichergestellt ist.

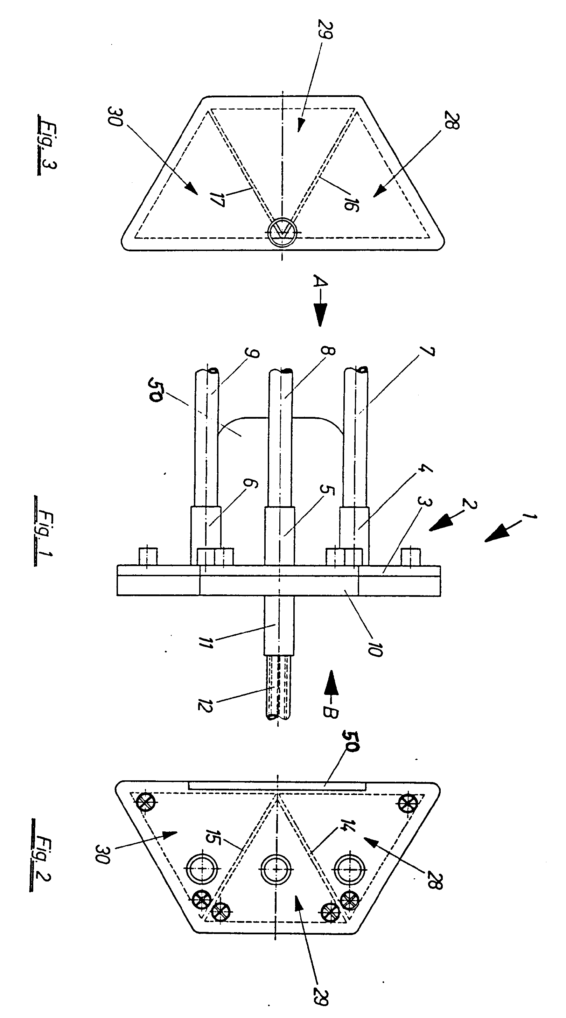

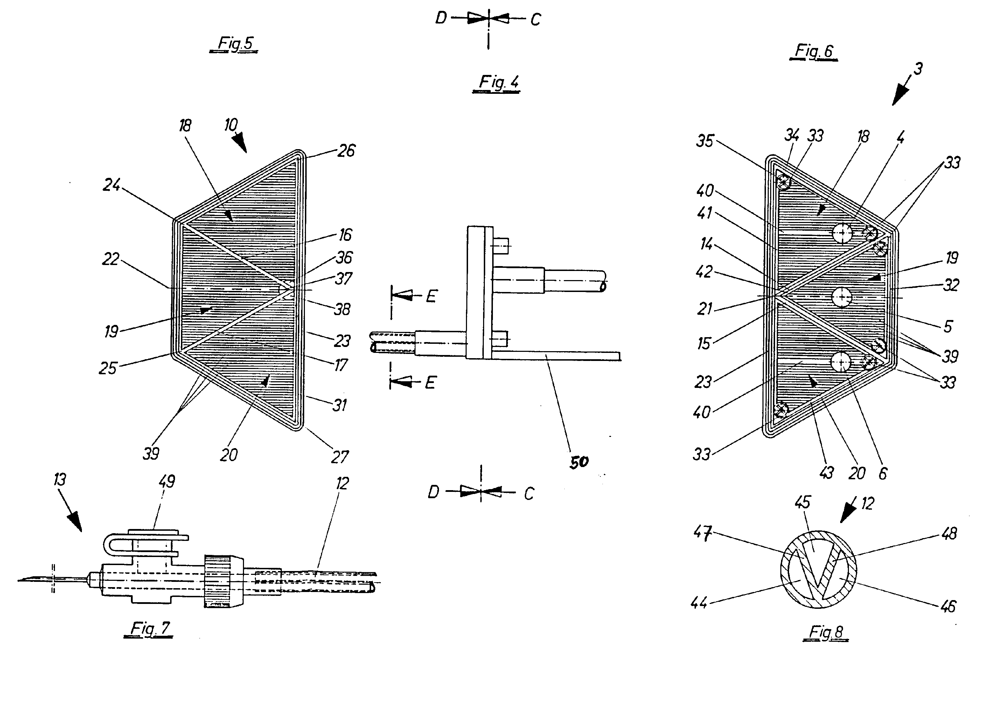

Den Figuren 1, 2 und 4 ist zu entnehmen, daß die Filtergehäusehälfte 3 mit einem sich senkrecht zu dieser Filtergehäusehälfte und damit auch den Filterelementen 28, 29 und 30 erstreckenden Auflageplatte 50 versehen ist, die im Bereich der Grundseite 23 mit der Filtergehäusehälfte 3 verbunden ist. Bei der Verwendung des Filters 1 ist anzustreben, daß die Plattenebene der Auflageplatte 50 horizontal ausgerichtet ist und entsprechend das Filtergehäuse 2 und die Filterelemente 28, 29 und 30 senkrecht, mit oben befindlichen infusions- bzw. injektionsseitigen Anschlußelementen 4, 5 und 6 und untenliegendem patientenseitigen Anschlußelement 11. Dies kann beispielsweise dadurch bewerkstelligt werden, daß das Filter 1 in einer entsprechenden Position am Bett des Patienten befestigt wird oder aber, um annäherungsweise diese Position zu gewährleisten, indem die Auflageplatte 50 entsprechend an einem im wesentlichen horizontal positionierten Unterarm des Patienten fixiert wird. In dieser Stellung fließen im Betrieb des Infusions- und/oder Injektionssystems die Flüssigkeiten separat über die Anschlußleitungen 7, 8 und 9 in die diesen jeweils zugeordnete Filterkammer 18, 19 bzw. 20 und verlassen diese nach wie vor voneinander getrennt durch die dem Anschlußelement 11 zugeordneten Kanäle 36, 37 und 38 und münden von dort in die diesen zugeordneten Kanäle 44, 45 bzw. 46 der patientenseitigen Anschlußleitung 12, in der die Flüssigkeiten nach wie vor getrennt geführt werden. Erst in dem sich an die Anschlußleitung 12 anschließenden patientennahen Anschlußstück 13, das beispielsweise als Kanüle mit einer Zuspritzstelle 49 ausgebildet sein kann, erfolgt die Vermischung der einzelnen Flüssigkeiten, somit in unmittelbarer Nähe des Patienten.

Von besonderer Bedeutung ist im Zusammenhang mit der Führung der Flüssigkeiten durch das Filter 1 die Ausbildung der Filtergehäusehälfte 3 mit dem senkrecht angeordneten Flüssigkeitsnuten 39 und 40 sowie den die Filterflächenbereiche umgebenden, die Form von Dreiecken aufweisenden Nuten 41, 42 und 43. So können die in den Flüssigkeiten befindlichen Partikel und Schwebeteilchen über die senkrechtstehenden Flüssigkeitsnuten 39 und 40 der Filtergehäusehälfte 3 in den jeweils der Grundseite 23 zugeordneten Bereich der Nuten 41, 42 und 43 absinken, wodurch gewährleistet wird, daß die Filterflächen der Filterelemente 28, 29 und 30 länger offen bleiben. Die umlaufenden Nuten 41, 42 und 43 werden aber gleichfalls als Luft- oder Gasleitnuten zu den Entlüftungsöffnungen 33 genutzt. Im übrigen fungieren die Flüssigkeitsnuten 39 beider Filtergehäusehälften 3 und 10 als Stützrippen für die blattartigen Filterelemente 28, 29 und 30.

Es ist vorgesehen, daß alle Teile des erfindungsgemäßen Infusions- und/oder Injektionssystems aus Kunststoffen bestehen, die eine thermoplastische Verbindung der einzelnen Systemteile, insbesondere der Einzelteile des Filters gestatten. Es liegt jedoch gleichfalls im Rahmen der vorliegenden Erfindung, die einzelnen Teile mittels Kleb- oder Klemmverbindungen miteinander zu verbinden, so kann beispielsweise die mehrkanalige patientenseitige Anschlußleitung im Übergangsbereich zur mehrkanaligen Austrittsfläche der Filterkammern mit dem diesen zugeordneten Anschlußelement verklebt sein, so daß eine nicht veränderbare Flüssigkeitsführung durch das Filter und die patientenseitige Anschlußleitung gewährleistet ist. Im übrigen kann das Material der Systemteile selbstverständlich variabel den verschiedenen chemischen notwendigen Beständigkeiten und biologischen Ansprüchen angepaßt werden.

## Patentansprüche

1. Infusions- und/oder Injektionssystem, mit einem Filter sowie infusions- und/oder injektionsseitigen und patientenseitigen Anschlußleitungen (7, 8, 9, 12),
   **gekennzeichnet** durch ein Filtergehäuse (2) mit mehreren separaten Filterkammern (18, 19, 20), die jeweils ein Filterelement (28, 29, 30) aufnehmen, ein dem Flüssigkeitseingang jeder Filterkammer (18, 19, 20) zugeordnetes Anschlußelement (4, 5, 6) zum Verbinden mit jeweils einer infusions- bzw. injektionsseitigen Anschlußleitung (7, 8, 9), sowie ein allen Filterkammern (18, 19, 20) gemeinsames Anschlußelement (11) zum Verbinden einer der Anzahl der Filterkammern (18, 19, 20) entsprechenden mehrkanaligen (Kanäle 44, 45, 46), patientenseitigen Anschlußleitung (12) mit den Flüssigkeitsausgängen (36, 37, 38) der Filterkammern (18, 19, 20), wobei diese Anschlußleitung (12) in ein einkanaliges, patientennahes Anschlußstück (13) mündet.

2. System nach Anspruch 1, dadurch gekennzeichnet, daß die Filterkammern (18, 19, 20) sich bezüglich des patientenseitigen Anschlußelementes (11) jeweils über einen Kreissektor erstrecken und mit ihren benachbarten, die Flüssigkeitsausgänge (36, 37, 38) aufweisenden Sektorspitzenbereichen in das patientenseitige Anschlußelement (11) münden, wobei die patientenseitige Anschlußleitung (12) entsprechend den Sektoren der Filterkammern (18, 19, 20) in einzelne Kanäle (44, 45, 46) unterteilt ist.

3. System nach Anspruch 1 oder 2, dadurch gekennzeichnet, daß das Filtergehäuse (2) die Form eines Trapezes aufweist, das in drei identisch ausgebildete, die Form von Dreiecken aufweisende Filterkammer (18, 19, 20) unterteilt ist, die unmittelbar aneinander angrenzend im Filtergehäuse (2) angeordnet ist.

**4.** System nach Anspruch 3, dadurch gekennzeichnet, daß die Dreiecke als gleichseitige Dreiecke ausgebildet sind.

**5.** System nach einem der Ansprüche 1 bis 4, dadurch gekennzeichnet, daß das Filtergehäuse (2) plattenförmig ausgebildet ist und mit diesem eine sich senkrecht zum Filtergehäuse (2) erstreckende Auflageplatte (50) verbunden ist.

**6.** System nach Anspruch 5, dadurch gekennzeichnet, daß das Filtergehäuse (2) die Form eines Trapezes aufweist und die Auflageplatte (50) mit der Grundseite (23) des Trapezes verbunden ist.

**7.** System nach einem der Ansprüche 1 bis 6, dadurch gekennzeichnet, daß jede Filterkammer (18, 19, 20) mit mindestens einer Entlüftungsöffnung (33) mit dieser zugeordneter Entlüftungsmembran (34) versehen ist.

**8.** System nach Anspruch 7, dadurch gekennzeichnet, daß die Filterkammern (18, 19, 20) die Form von Dreiecken aufweisen und jede Filterkammer (18, 19, 20) im Bereich der beiden der patientenseitigen Anschlußleitung (12) abgewandten Ecken (26, 24, 24, 25; 25, 27) eine Entlüftungsöffnung (33) mit einer in diese eingesetzten hydrophoben Membran (34) aufweist.

**9.** System nach einem der Ansprüche 1 bis 8, dadurch gekennzeichnet, daß das Filtergehäuse (2) benachbart zur Auflageplatte (50) das Anschlußelement (11) zum Verbinden mit der patientenseitigen Anschlußleitung (12) und beabstandet zur Auflageplatte (50) die Anschlußelemente (4, 5, 6) zum Verbinden mit den infusions- bzw. injektionsseitigen Anschlußleitungen (7, 8, 9) aufweist.

**10.** System nach Anspruch 9, dadurch gekennzeichnet, daß die Anschlußelemente (4, 5, 6) zum Verbinden mit dem infusions- bzw. injektionsseitigen Anschlußleitungen (7, 8, 9) in gleichem Abstand zur Auflageplatte (50) angeordnet sind.

**11.** System nach einem der Ansprüche 5 bis 10, dadurch gekennzeichnet, daß das Filtergehäuse (2) aus zwei in einer Ebene senkrecht zur Auflageplatte (50) geteilten Filtergehäusehälften (3, 10) besteht, wobei eine (3) der beiden Filtergehäusehälften (3, 10) die Anschlußelemente (4, 5, 6) zum Verbinden mit den infusions- und/oder injektionsseitigen Anschlußleitungen (7, 8, 9), die andere Filtergehäusehälfte (10) das Anschlußelement (11) zum Verbinden mit der patientenseitigen Anschlußleitung (12) aufweist.

**12.** System nach Anspruch 11, dadurch gekennzeichnet, daß die Filtergehäusehälften (3, 10) außen miteinander verschweißt sind und die Filterkammern (18, 19, 20) durch Trennstege (14, 15; 16, 17) gebildet sind.

**13.** System nach Anspruch 11 oder 12, dadurch gekennzeichnet, daß die den infusions- bzw. injektionsseitigen Anschlußelementen (4, 5, 6) zugeordnete Filtergehäusehälfte (3) im Bereich der Filterelemente (28, 29, 30) jeweils eine diese umgebende umlaufende Nut (41, 42, 43) aufweist.

**14.** System nach einem der Ansprüche 11 bis 13, dadurch gekennzeichnet, daß die Filtergehäusehälften (3, 10) Stützrippen (39, 40) für die Filterelemente (28, 29, 30) aufweisen, insbesondere Stützrippen, die senkrecht zur Ebene der Auflageplatte (50) angeordnet sind.

**15.** System nach einem der Ansprüche 1 bis 14, dadurch gekennzeichnet, daß es aus Kunststoff besteht.

## Claims

**1.** Infusion and/or injection system, with a filter and also with connecting conduits (7, 8, 9, 12) at the infusion and/or injection side and at the patient side, characterised by a filter housing (2) with a plurality of separate filter chambers (18, 19, 20) which each accommodate a filter element (28, 29, 30), a connecting element (4, 5, 6) associated with the liquid inlet of each filter chamber (18, 19, 20) and intended for connection to in each case a connecting conduit (7, 8, 9) at the infusion or injection side, and also a connecting element (11) common to all the filter chambers (18, 19, 20) and intended for the connecting of a multi-duct (ducts 44, 45, 46) patient-side connecting conduit (12), the number of ducts corresponding to the number of filter chambers (18, 19, 20), to the liquid outlets (36, 37, 38) of the filter chambers (18, 19, 20), the said connecting conduit (12) opening into a single-duct connecting element (13) near the patient.

**2.** System according to claim 1, characterised in that the filter chambers (18, 19, 20) extend in each case over a sector of a circle in regard to the connecting element (11) situated at the patient side, and, with their adjacent sector apex regions, which comprise the liquid outlets (36, 37, 38), open into the patient-side connecting element (11), the patient-side connecting conduit (12) being sub-divided into individual ducts (44, 45, 46) corresponding to the sectors of the filter chambers (18, 19, 20).

3. System according to claim 1 or 2, characterised in that the form of the filter housing (2) is trapezoidal, sub-divided into three identically formed filter chambers (18, 19, 20) in the form of triangles, the chambers being situated directly adjoining one another in the filter housing (2).

4. System according to claim 3, characterised in that the triangles are constructed as equilateral triangles.

5. System according to one of claims 1 to 4, characterised in that the filter housing (2) is plate-shaped, and a supporting-surface plate (50) extending at right angles to the filter housing (2) is connected to the said housing.

6. System according to claim 5, characterised in that the filter housing (2) is trapezoidal in form, and the supporting-surface plate (50) is connected to the base side (23) of the trapezoid.

7. System according to one of claims 1 to 6, characterised in that each filter chamber (18, 19, 20) is provided with at least one venting aperture (33) with a venting membrane (34) associated therewith.

8. System according to claim 7, characterised in that the filter chambers (18, 19, 20) are in the form of triangles, and each filter chamber (18, 19, 20) has in the region of the two corners (26, 24, 24, 25; 25, 27) remote from the patient-side connecting conduit (12) a venting aperture (33) with a hydrophobic membrane (34) inserted in the said aperture.

9. System according to one of claims 1 to 8, characterised in that the filter housing (2) comprises adjacent the supporting-surface plate (50) the connecting element (11) for connection to the patient-side connecting conduit (12), and at a spacing from the supporting-surface plate (50) the connecting elements (4, 5, 6) for connection to the connecting conduits (7, 8, 9) situated at the infusion or injection side.

10. System according to claim 9, characterised in that the connecting elements (4, 5, 6) for connection to the connecting conduits (7, 8, 9) situated at the infusion or injection side are situated at equal spacings from the supporting-surface plate (50).

11. System according to one of claims 5 to 10, characterised in that the filter housing (2) is made of two filter housing halves (3, 10) divided in a plane at right angles to the supporting-surface plate (50), one (3) of the two filter housing halves (3, 10) comprising the connecting elements (4, 5, 6) for connection to the connecting conduits (7, 8, 9) situated at the infusion and/or injection side, and the other filter housing half (10) comprising the connecting element (11) for connection to the connecting conduit (12) situated at the patient side.

12. System according to claim 11, characterised in that the filter housing halves (3, 10) are welded to one another externally, and the filter chambers (18, 19, 20) are formed by partition webs or strips (14, 15; 16, 17).

13. System according to claim 11 or 12, characterised in that the filter housing half (3) associated with the connecting elements (4, 5, 6) situated at the infusion or injection side has in the region of each filter element (28, 29, 30) an encircling groove (41, 42, 43) which surrounds same.

14. System according to one of claims 11 to 13, characterised in that the filter housing halves (3, 10) comprise supporting ribs (39, 40) for the filter elements ( 28, 29, 30), especially supporting ribs which are arranged at right angles to the plate of the supporting-surface plate (50).

15. System according to one of claims 1 to 14, characterised in that it is made of synthetic plastic material.

**Revendications**

1) Système d'infusion et/ou d'injection comprenant un filtre ainsi que des conduits de raccordement (7,8,9,12) du côté de l'infusion et/ou de l'injection et du côté patient, caractérisé par un boîtier de filtre (2) à plusieurs chambres séparées (18,19,20), qui reçoivent chacune un élément de filtre (28,29,30), un élément de liaison (4,5,6) associé à l'entrée du liquide de chaque chambre de filtre (18,19,20) destiné à l'emboîtement respectivement d'un conduit de raccordement (7,8,9) du côté de l'infusion ou injection, ainsi qu'un élément de liaison (11) commun à toutes les chambres à filtre (18,19,20), destiné à emboîter le conduit de raccordement (12) du côté du patient, à plusieurs canaux (canaux 44,45,46) correspondant au nombre des chambres à filtre (18,19,20), dans les sorties de liquide (36,37,38) des chambres à filtre (18,19,20), ce conduit de raccordement (12) débouchant dans une pièce de jonction (13) à un seul canal, située du côté du patient.

2) Système selon la revendication 1, caractérisé en ce que les chambres à filtre (18,19,20) s'étendent chacune sur un secteur circulaire par rapport à l'élément de liaison (11) du côté du patient et débouchent dans l'élément de liaison (11) du côté du patient au niveau des pointes des secteurs comprenant les sorties de liquide (36,37,38), le conduit de raccordement (12) du côté du patient étant subdivisé en canaux particuliers (44,45,46) correspondant aux secteurs des chambres à filtre (18,19,20).

3) Système selon la revendication 1 ou la revendication 2, caractérisé en ce que le boîtier de filtre (2) à la forme d'un trapèze, subdivisé en trois chambres à filtre (18,19,20) de forme triangulaire identique, qui sont disposées dans le boîtier de filtre (2) directement l'une à côté de l'autre.

4) Système selon la revendication 3, caractérisé en ce que les triangles sont des triangles équilatéraux.

5) Système selon une des revendications 1 à 4, caractérisé en ce que le boîtier de filtre (2) a une forme de plaque et une plaque d'appui (50) perpendiculaire au boîtier du filtre (2) lui est reliée.

6) Système selon la revendication 5, caractérisé en ce que le boîtier de filtre (2) a la forme d'un trapèze et la plaque d'appui (50) est reliée à la base (23) du trapèze.

7) Système selon une des revendications 1 à 6, caractérisé en ce que chaque chambre à filtre (18,19,20) est munie d'au moins une ouverture de désaération (33) avec une membrane de purge d'air (34) associée à celle-ci.

8) Système selon la revendication 7, caractérisé en ce que les chambres à filtre (18,19,20) ont une forme de triangle et chaque chambre à filtre (18,19,20) comprend au niveau de ses deux angles (26,24 ; 24,25 ; 25,27) opposés au conduit de raccordement (12) du côté du patient une ouverture de désaération (33) équipée d'une membrane hydrophobe.

9) Système selon une des revendications 1 à 8, caractérisée en ce que le boîtier de filtre (2) a au voisinage de la plaque d'appui 50 l'élément de liaison (11) destiné à emboîter le conduit de raccordement (12) du côté du patient et à une certaine distance de la plaque d'appui (50) les éléments de liaison (4,5,6) destinés à l'emboîtement des conduits de raccordement (7,8,9) du côté de l'infusion ou injection.

10) Système selon la revendication 9, caractérisé en ce que les éléments de liaison (4,5,6) destinés à emboîter les conduits de raccordement (7,8,9) du côté de l'infusion ou injection sont placés à la même distance de la plaque d'appui (50).

11) Système selon une des revendications 5 à 10, caractérisé en ce que le boîtier de filtre (2) se compose de deux moitiés de boîtier (3,10) séparées dans un plan perpendiculaire à la plaque d'appui (50), et une (3) des deux moitiés de boîtier (3,10) comporte les éléments de liaison (4,5,6) pour l'emboîtement des conduits de raccordement (7,8,9) du côté de l'infusion et/ou de l'injection, tandis que l'autre moitié de filtre (10) comporte l'élément de liaison (11) pour l'emboîtement du conduit de raccordement (12) du côté du patient.

12) Système selon la revendication 11, caractérisé en ce que les moitiés du boîtier de filtre (3,10) sont assemblées par sondage extérieur et les chambres de filtre (18,18,20) sont formées par des entretoises de séparation (14,15 ; 16,17).

13) Système selon la revendication 11 ou la revendication 12, caractérisé en ce que la moitié du boîtier de filtre (3) associée aux éléments de liaison (4,5,6) du côté de l'infusion ou injection a au niveau des éléments de filtrage (28,28,30) respectivement une rainure (41,42,43) circulante entourant chacun d'eux.

14) Système selon une des revendications 11 à 13, caractérisé en ce que les moitiés de boîtier de filtre (3,10) ont des nervures d'appui (39,40) pour les éléments de filtrage (28,29,30), en particulier des nervures d'appui, qui sont placées perpendiculairement à la plaque d'appui (50).

15) Système selon une des revendications 1 à 14, caractérisé en ce qu'il est constitué d'une matière plastique.

Fig. 3

Fig. 1

Fig. 2

Fig. 6

Fig. 8

Fig. 4

Fig. 5

Fig. 7